Europäisches Patentamt

European Patent Office

Office européen des brevets

(11) Numéro de publication: **0 131 081**
**A1**

(12) # DEMANDE DE BREVET EUROPEEN

(21) Numéro de dépôt: **83401428.4**

(22) Date de dépôt: **11.07.83**

(51) Int. Cl.⁴: **C 09 J 7/04**
**A 61 L 15/06**

(43) Date de publication de la demande:
**16.01.85 Bulletin 85/3**

(84) Etats contractants désignés:
**AT BE CH DE GB IT LI LU NL**

(71) Demandeur: **Mechin, Jean-Claude**
**109 boulevard Malesherbes**
**F-75008 Paris(FR)**

(72) Inventeur: **Mechin, Jean-Claude**
**109 boulevard Malesherbes**
**F-75008 Paris(FR)**

(74) Mandataire: **L'Helgoualch, Jean et al,**
**OFFICE PICARD 134 Boulevard de Clichy**
**F-75018 Paris(FR)**

(54) **Produit autocollant, procédé pour sa fabrication et application comme pansement, feuille d'emballage ou ruban de fixation.**

(57) L'invention concerne un nouveau produit autocollant sous forme de feuille, bande ou ruban.

Il comprend un support alvéolaire enduit d'une couche d'élastomère synthétique ou naturel préalablement traité par chauffage à une température comprise entre 70 et 160°C, puis refroidissement.

Application comme pansement, feuille d'emballage ou ruban de fixation.

EP 0 131 081 A1

- 1 -

## Produit autocollant, procédé pour sa fabrication et application comme pansement, feuille d'emballage ou ruban de fixation

La présente invention concerne un nouveau dispositif d'attache, et plus particulièrement un nouveau produit autocollant sous forme de feuille, de bande ou de ruban, un procédé pour sa fabrication, ainsi que son application comme pansement, feuille d'emballage et ruban de fixation.

Dans le domaine des pansements et de l'emballage, notamment, on connait divers produits autocollants présentés généralement sous forme de rubans. Ces rubans adhèrent à la plupart des surfaces avec lesquelles ils peuvent être mis en contact, ce qui constitue souvent un inconvénient. Au contraire, les produits non adhésifs utilisés par exemple pour le ficelage des colis ou paquets, doivent être fixés par agrafage ou nouage, ce qui complique leur mode d'utilisation et occasionne des pertes de temps. On connait par ailleurs des rubans autocollants dont la surface peut être rendue adhésive par humidification, mais la force d'adhérence est généralement insuffisamment élevée.

La présente invention a pour objet un produit autocollant en forme de feuille, de bande ou de ruban dont la mise en place peut être faite rapidement et facilement, sans qu'il soit nécessaire d'utiliser aucun accessoire.

L'invention a aussi pour objet l'application d'un nouveau produit autocollant comme feuille d'emballage, ruban de fixation ou pansement.

L'invention concerne également un procédé permettant de fabriquer un nouveau produit autocollant par enduction d'un élastomère synthétique ou naturel sur un support alvéolaire.

Le produit autocollant conforme à l'invention, du type comportant un support enduit d'au moins une couche autocollante, est constitué par un support alvéolaire enduit d'une couche d'élastomère synthétique ou naturel, préalablement traité par chauffage à une température comprise entre 70 et 160°C, puis refroidissement, conformément au procédé décrit ci-après.

Le support peut être constitué par toute feuille ou ruban de matière alvéolaire, et par exemple par une bande de gaze, ou une feuille souple en matière synthétique ou naturelle comportant des perforations. La dimension des alvéoles du support est inférieure à 1cm environ, et de préférence comprise entre 1 et 3mm, mais elle peut être encore inférieure.

L'élastomère enduit sur le support est un élastomère synthétique ou naturel, et par exemple un caoutchouc naturel disponible dans le commerce sous forme de crèpe pâle, crèpe brun ou feuille fumée, selon la qualité. Suivant l'invention, on utilise de préférence le caoutchouc naturel de qualité crèpe pâle.

On peut également utiliser un élastomère synthétique et notamment du cis-polyisoprène-1,4, un copolymère butadiène-styrène, un cis-polybutadiène-1,4, un polyoxypropylène, un polyoxychloropropylène, un polyacrylate, etc. ou encore un dérivé ou un polymère ou copolymère greffé. Suivant l'invention on utilise de préférence un copolymère butadiène-styrène obtenu par polymérisation du butadiène et du styrène en présence d'un catalyseur au persulfate, le cis-polyisoprène-1,4 obtenu par polymérisation stéréospécifique en présence d'un catalyseur à base de tétrachlorure de titane et de triéthyl-aluminium ou de triéthyl-lithium (catalyseur de type Ziegler-Natta), ou encore le cis-polybutadiène-1,4 obtenu par polymérisation stéréospécifique en présence de triéthyl-aluminium et d'un sel de cobalt ou de tétraiodure de titane.

Le cas échéant, l'élastomère peut être coloré en masse par addition d'un colorant. Un motif figuratif ou une inscription peut également être apposé sur chacune des faces par impression.

Conformément au procédé de l'invention, l'élastomère est chauffé pendant une durée de 30mn à 2 heures environ, à une température comprise entre 70 et 160°C, et de préférence entre 80 et 100°C, on le laisse refroidir jusqu'à une température inférieure à 50°C environ, puis on le plonge dans un solvant approprié que l'on laisse reposer pendant au moins 6 heures, et on procède ensuite à un malaxage pour obtenir un produit pâteux que l'on enduit sur le support.

La température à laquelle est chauffé l'élastomère est de préférence de l'ordre de 80 à 100°C comme indiqué ci-dessus, mais elle peut varier en fonction de la nature et de la pureté de l'élastomère utilisé. Si la température est trop élevée les chaînes polymères sont détruites et le produit se dégrade, tandis que si la température n'est pas assez élevée la dissolution dans le solvant est insuffisante.

Il est avantageux que l'élastomère soit sous forme de plaques ou de feuilles, que l'on utilise à raison de 5 à 50g environ et de préférence 10 à 30g pour 1 litre de solvant.

Le solvant peut être un solvant organique tel qu'un solvant aliphatique, et par exemple l'éther diéthylique ou l'éther diméthylique, ou un solvant aromatique et par exemple le phénol, le naphtol, le benzène, le toluène, etc; on peut également utiliser un solvant chloré tel que le tétrachlorure de carbone ou le chloroforme.

La pâte obtenue après malaxage, pendant une durée d'environ 5 à 20 minutes, est étalée sur le support par les techniques usuelles, puis on procède à un séchage, par exemple par passage d'air chaud, pour évaporer le solvant restant. L'enduction peut par exemple être effectuée manuellement au moyen d'une raclette, ou mécaniquement en faisant défiler le support, par exemple un ruban de gaze, dans un bac contenant la pâte, puis entre deux rouleaux presseurs qui permettent d'ajuster l'épaisseur de la couche.

L'épaisseur de la couche après séchage peut être limitée si on le désire à une valeur faible, inférieure à 1mm.

Lorsqu'elle est enduite, la feuille ou la bande servant de support est revêtue d'une feuille intercalaire, en papier, par exemple un papier gaufré ou mince, puis elle est enroulée sur elle-même pour être stockée. Le produit autocollant enduit n'adhère pas au papier intercalaire, et peut

- 4 -

être conservé à plat ou en rouleau pendant une période prolongée.

Le produit autocollant conforme à la présente invention présente l'avantage de n'adhérer à pratiquement aucun autre produit que lui-même. Il peut donc être utilisé dans diverses applications où il est nécessaire de disposer d'un produit enveloppant susceptible d'être maintenu en place sans coller aux parties avec lesquelles il est en contact.

Le produit autocollant suivant l'invention peut notamment être utilisé en médecine humaine ou vétérinaire comme pansement ou comme bandage appliqué par exemple sur un membre ou un doigt, ou sur une patte d'un animal. Il est tout particulièrement avantageux dans une telle application car il est chimiquement neutre et n'adhère ni à la peau, ni aux poils, ni à la plaie.

De plus, on peut incorporer dans la couche d'élastomère, ou appliquer à sa surface, diverses substances médicamenteuses, telles que antihémorragiques, désinfectants, calmants, antibiotiques, sans altérer ses caractéristiques physiques et chimiques. On ne peut toutefois utiliser un tel pansement sur une surface non fermée.

Le produit autocollant suivant l'invention peut également être utilisé dans le domaine de l'emballage, comme feuille d'emballage destinée par exemple à envelopper des colis sans aucun dispositif accessoire de fixation. Des indications nécessaires à l'identification du colis ou des motifs figuratifs peuvent être appliqués par impression directe ou sous forme d'étiquettes collées sur sa surface.

Il peut également être utilisé sous forme de ruban de fixation du type formant au moins une boucle, pour le ficelage d'objets fragiles auxquels il ne doit pas adhérer, tels que des tissus ou des vêtements; son caractère autocollant permet une attache immédiate par simple contact sur lui-même lorsqu'il forme une boucle, ce qui évite l'obligation de le fixer par un noeud, par collage ou agrafage. De plus sa grande puissance autocollante garantit contre tout risque d'arrachement en cours de manipulation, et il ne laisse aucune trace sur les objets avec lesquels il peut se trouver en contact. On peut notamment l'utiliser dans des dispositifs dérouleurs-coupeurs couramment employés pour les rubans adhésifs.

A titre d'exemple non limitatif, on décrit ci-après la préparation d'une bande autocollante utilisable comme pansement.

## EXEMPLE

Dans une étuve, on chauffe 300g de cis-polyisoprène-1,4 sous forme de feuilles minces de quelques millimètres d'épaisseur pendant 90mn à 90°C.

On sort le cis-polyisoprène de l'étuve, on le laisse refroidir jusqu'à environ 25°C, on l'ajoute à 20 l d'éther diéthylique puis on laisse reposer pendant environ 12 heures. On procède ensuite à un malaxage pendant environ 15mn, jusqu'à obtenir un produit pâteux, auquel on ajoute un antiseptique. La pâte est enduite sur une gaze, à raison de 500g environ par m2 de gaze, puis on sèche sous courant d'air chaud à 22°C pendant 2mn.

L'épaisseur à sec de la couche enduite est d'environ 0,5mm. L'ensemble est ensuite découpé en bandes pour constituer des pansements.

REVENDICATIONS

1.   Nouveau produit autocollant sous forme de feuille, de bande ou de ruban comportant un support enduit d'au moins une couche autocollante, caractérisé en ce qu'il comprend un support alvéolaire enduit d'une couche d'élastomère synthétique ou naturel préalablement traité par chauffage à une température comprise entre 70 et 160°C, puis refroidissement.

2.   Produit autocollant selon la revendication 1, caractérisé en ce que l'élastomère est un caoutchouc naturel ou du cis-polyisoprène-1,4, un copolymère butadiène-styrène, un cis-polybutadiène-1,4, un polyoxypropylène, un polyoxychloropropylène ou un polyacrylate.

3.   Produit autocollant selon l'une quelconque des revendications 1 et 2, caractérisé en ce que le support est constitué par une bande de gaze.

4.   Pansement caractérisé en ce qu'il est constitué par un produit autocollant selon l'une quelconque des revendications 1 à 3, présenté sous forme de bande.

5.   Pansement selon la revendication 4, caractérisé en ce que la couche d'élastomère est imprégnée par un principe actif de médicament.

6.   Feuille d'emballage caractérisée en ce qu'elle est constituée par un produit autocollant selon l'une quelconque des revendications 1 à 3, présenté sous forme de feuille.

7.   Ruban de fixation du type formant au moins une boucle, caractérisé en ce qu'il est constitué par un produit autocollant selon l'une quelconque des revendications 1 à 3, présenté sous forme de ruban.

8.   Procédé pour la fabrication d'un produit autocollant sous forme de feuille, de bande ou de ruban, comportant un support alvéolaire enduit d'au moins une couche autocollante, caractérisé en ce qu'on chauffe un élastomère synthétique ou naturel à une température comprise entre 70 et 160°C, on refroidit à une température inférieure à 50°C, on le plonge dans un solvant que l'on laisse reposer, puis on procède à un malaxage pour obtenir un produit pâteux que l'on enduit sur le support.

9.   Procédé selon la revendication 8, caractérisé en ce que l'élastomère est un caoutchouc naturel ou du cis-polyisoprène-1,4, un copolymère butadiène-styrène, un cis-polybutadiène-1,4, un polyoxypropylène, un polyoxychloropropylène ou un polyacrylate.

10. Procédé selon l'une quelconque des revendications 8 et 9, caractérisé en ce que l'élastomère est chauffé à une température comprise entre 80 et 100°C.

11. Procédé selon la revendication 8, caractérisé en ce que le solvant est l'éther diéthylique, l'éther diméthylique, le phénol, le naphtol, le benzène, le toluène, le tétrachlorure de carbone ou le chloroforme.

12. Procédé selon la revendication 8, caractérisé en ce qu'on utilise 10 à 30g d'élastomère par litre de solvant.

**0131081**
Numero de la demande

**RAPPORT DE RECHERCHE EUROPEENNE**

Office européen
des brevets

EP 83 40 1428

## DOCUMENTS CONSIDERES COMME PERTINENTS

| Catégorie | Citation du document avec indication en cas de besoin des parties pertinentes | Revendication concernée | CLASSEMENT DE LA DEMANDE (Int Cl ³) |
|---|---|---|---|
| A | FR-A-1 534 424 (JOHNS-MANVILLE) <br> * page 7, résumé C; page 2, colonne de droite, lignes 49-55 * | 1 | C 09 J 7/04 <br> A 61 L 15/06 |
| A | DE-B-1 032 454 (MINNESOTA MINING) <br> * Colonne 1, lignes 1-10; colonne 4, exemple 1, lignes 46-69 * | 1 | |
| A | FR-A- 953 239 (MINNESOTA MINING) <br> * Page 1, lignes 1-34 * | 1 | |

**DOMAINES TECHNIQUES RECHERCHES (Int. Cl. ³)**

C 09 J
A 61 L

Le présent rapport de recherche a été établi pour toutes les revendications

| Lieu de la recherche | Date d'achèvement de la recherche | Examinateur |
|---|---|---|
| LA HAYE | 02-03-1984 | GIRARD Y.A. |